# EUROPEAN PATENT SPECIFICATION

(11) **EP 3 285 767 B1**
(45) Date of publication and mention of the grant of the patent: **20.03.2019**
(21) Application number: 16718885.3
(22) Date of filing: 22.04.2016
(51) Int. Cl.: A61K 31/437, A61P 21/00

(54) **TREATMENT OF PAIN**
BEHANDLUNG VON SCHMERZEN
TRAITEMENT DE LA DOULEUR

(30) Priority: 24.04.2015 GB 201507048
(43) Date of publication of application: 28.02.2018
(73) Proprietor: BenevolentAI Cambridge Limited, London NW1 1LW (GB)
(72) Inventor: GONZALEZ, Isabel, Cambridge Cambridgeshire CB22 3AT (GB); PRITCHARD, Martyn, Cambridge Cambridgeshire CB22 3AT (GB); RICHARDSON, Peter, Cambridge Cambridgeshire CB22 3AT (GB)
(74) Representative: Gill Jennings & Every LLP
(86) International application number: PCT/GB2016/051122
(87) International publication number: WO 2016/170353

(56) References cited:
- WO-A1-2010/031789
- WO-A1-2014/199171
- WO-A1-2015/159112
- US-A1- 2007 066 646

## Description

### Field of the Invention

The present invention relates to the use of the SSAO inhibitor (3*S*)-Tetrahydrofuran-3-yl (4*S*)-4-isopropyl-1,4,6,7-tetrahydro-5H-imidazo[4,5-c]pyridine-5-carboxylate, and salts thereof in the treatment of pain.

### Background of the Invention

Semicarbazide-sensitive amine oxidase (SSAO) activity is an enzyme activity expressed by Vascular Adhesion Protein-1 (VAP-1) or Amine Oxidase, Copper Containing 3 (AOC3), belongs to the copper-containing amine oxidase family of enzymes (EC.1.4.3.6). Therefore inhibitors of the SSAO enzyme may also modulate the biological functions of the VAP-1 protein.

SSAO activity has been found in a variety of tissues including vascular and non-vascular smooth muscle tissue, endothelium, and adipose tissue [Lewinsohn, Braz. J. Med. Biol. Res. 1984, 17, 223-256; Nakos & Gossrau, Folia Histochem. Cytobiol. 1994, 32, 3-10; Yu et al., Biochem. Pharmacol. 1994, 47, 1055-1059; Castillo et al., Neurochem. Int. 1998, 33, 415-423; Lyles & Pino, J. Neural. Transm. Suppl. 1998, 52, 239-250; Jaakkola et al., Am. J. Pathol. 1999, 155, 1953-1965; Morin et al., J. Pharmacol. Exp. Ther. 2001, 297, 563-572; Salmi & Jalkanen, Trends Immunol. 2001, 22, 211-216]. In addition, SSAO protein is found in blood plasma and this soluble form appears to have similar properties as the tissue-bound form [Yu et al., Biochem. Pharmacol. 1994, 47, 1055-1059; Kurkijärvi et al., J. Immunol. 1998, 161, 1549-1557].

The precise physiological role of this abundant enzyme has yet to be fully determined, but it appears that SSAO and its reaction products may have several functions in cell signalling and regulation. For example, recent findings suggest that SSAO plays a role in both GLUT4-mediated glucose uptake [Enrique-Tarancon et al., J. Biol. Chem. 1998, 273, 8025-8032; Morin et al., J. Pharmacol. Exp. Ther. 2001, 297, 563-572] and adipocyte differentiation [Fontana et al., Biochem. J. 2001, 356, 769-777; Mercier et al., Biochem. J. 2001, 358, 335-342]. In addition, SSAO has been shown to be involved in inflammatory processes where it acts as an adhesion protein for leukocytes [Salmi & Jalkanen, Trends Immunol. 2001, 22, 211-216; Salmi & Jalkanen, in "Adhesion Molecules: Functions and Inhibition" K. Ley (Ed.), 2007, pp. 237-251], and might also play a role in connective tissue matrix development and maintenance [Langford et al., Cardiovasc. Toxicol. 2002, 2(2), 141-150; Göktürk et al., Am. J. Pathol. 2003, 163(5), 1921-1928]. Moreover, a link between SSAO and angiogenesis has recently been discovered [Noda et al., FASEB J. 2008, 22(8), 2928-2935], and based on this link it is expected that inhibitors of SSAO have an anti-angiogenic effect.

Several studies in humans have demonstrated that SSAO activity in blood plasma is elevated in conditions such as congestive heart failure, diabetes mellitus, Alzheimer's disease, and inflammation [Lewinsohn, Braz. J. Med. Biol. Res. 1984, 17, 223-256; Boomsma et al., Cardiovasc. Res. 1997, 33, 387-391; Ekblom, Pharmacol. Res. 1998, 37, 87-92; Kurkijärvi et al., J. Immunol. 1998, 161, 1549-1557; Boomsma et al., Diabetologia 1999, 42, 233-237; Meszaros et al., Eur. J. Drug Metab. Pharmacokinet. 1999, 24, 299-302; Yu et al., Biochim. Biophys. Acta 2003, 1647(1-2), 193-199; Mátyus et al., Curr. Med. Chem. 2004, 11(10), 1285-1298; O'Sullivan et al., Neurotoxicology 2004, 25(1-2), 303-315; del Mar Hernandez et al., Neurosci. Lett. 2005, 384(1-2), 183-187]. It has been suggested that reactive aldehydes and hydrogen peroxide produced by endogenous amine oxidases contribute to the progression of cardiovascular diseases, diabetic complications and Alzheimer's disease [Callingham et al., Prog. Brain Res. 1995, 106, 305-321; Ekblom, Pharmacol. Res. 1998, 37, 87-92; Yu et al., Biochim. Biophys. Acta 2003, 1647(1-2), 193-199; Jiang et al., Neuropathol Appl Neurobiol. 2008, 34(2), 194-204]. Furthermore, the enzymatic activity of SSAO is involved in the leukocyte extravasation process at sites of inflammation where SSAO has been shown to be strongly expressed on the vascular endothelium [Salmi et al., Immunity 2001, 14(3), 265-276; Salmi & Jalkanen, in "Adhesion Molecules: Functions and Inhibition" K. Ley (Ed.), 2007, pp. 237-251]. Accordingly, inhibition of SSAO has been suggested to have a therapeutic value in the prevention of diabetic complications and in inflammatory diseases [Ekblom, Pharmacol. Res. 1998, 37, 87-92; Salmi et al., Immunity 2001, 14(3), 265-276; Salter-Cid et al., J. Pharmacol. Exp. Ther. 2005, 315(2), 553-562].

WO2007/146188 teaches that blocking SSAO activity inhibits leucocyte recruitment, reduces the inflammatory response, and is expected to be beneficial in prevention and treatment of seizures, for example, in epilepsy.

O'Rourke et al (J Neural Transm. 2007;114(6):845-9) examined the potential of SSAO inhibitors in neurological diseases, having previously demonstrated the efficacy of SSAO inhibition in a rat model of stroke. An SSAO inhibitor is tested on relapsing-remitting experimental autoimmune encephalomyelitis (EAE), a mouse model that shares many characteristics with human multiple sclerosis. The data demonstrates the potential clinical benefit of small molecule anti-SSAO therapy in this model and therefore in treatment of human multiple sclerosis.

SSAO knockout animals are phenotypically overtly normal but exhibit a marked decrease in the inflammatory responses evoked in response to various inflammatory stimuli [Stolen et al., Immunity 2005, 22(1), 105-115]. In addition, antagonism of its function in wild type animals in multiple animal models of human disease (e.g. carrageenan-induced paw inflammation, oxazolone-induced colitis, lipopolysaccharide-induced lung inflammation, collagen-induced arthritis, endotoxin-induced uveitis) by the use of antibodies and/or small molecules has been shown to be protective in decreasing the leukocyte infiltration, reducing the severity of the disease phenotype and reducing levels of inflammatory cytokines and chemokines [Kirton et al., Eur. J. Immunol. 2005, 35(11), 3119-3130; Salter-Cid et al., J. Pharmacol. Exp. Ther. 2005, 315(2), 553-562; McDonald et al., Annual Reports in Medicinal Chemistry 2007, 42, 229-243; Salmi & Jalkanen, in "Adhesion Molecules: Functions and Inhibition" K. Ley (Ed.), 2007, pp. 237-251; Noda et al., FASEB J. 2008 22(4), 1094-1103; Noda et al., FASEB J. 2008, 22(8), 2928-2935]. This anti-inflammatory protection seems to be afforded across a wide range of inflammatory models all with independent causative mechanisms, rather than being restricted to one particular disease or disease model. This would suggest that SSAO may be a key nodal point for the regulation of the inflammatory response, and it is therefore likely that SSAO inhibitors will be effective anti-inflammatory drugs in a wide range of human and animal diseases. VAP-1 has also been implicated in the progression and maintenance of fibrotic diseases including those of the liver and lung. Weston and Adams (J Neural Transm. 2011, 118(7), 1055-64) have summarised the experimental data implicating VAP-1 in liver fibrosis, and Weston et al (EASL Poster 2010) reported that blockade of VAP-1 accelerated the resolution of carbon tetrachloride induced fibrosis. In addition VAP-1 has been implicated in inflammation of the lung (e.g. Singh et al., 2003, Virchows Arch 442:491-495) suggesting that VAP-1 blockers would reduce lung inflammation and thus be of benefit to the treatment of cystic fibrosis by treating both the pro-fibrotic and pro-inflammatory aspects of the disease.

SSAO (VAP-1) is up regulated in gastric cancer and has been identified in the tumour vasculature of human melanoma, hepatoma and head and neck tumours (Yoong KF, McNab G, Hubscher SG, Adams DH. (1998), J Immunol 160, 3978-88.; Irjala H, Salmi M, Alanen K, Gre'nman R, Jalkanen S (2001), Immunol. 166, 6937-6943; Forster-Horvath C, Dome B, Paku S, et al. (2004), Melanoma Res. 14, 135-40.). One report (Marttila-Ichihara F, Castermans K, Auvinen K, Oude Egbrink MG, Jalkanen S, Griffioen AW, Salmi M. (2010), J Immunol. 184, 3164-3173.) has shown that mice bearing enzymically inactive VAP-1 grow melanomas more slowly, and have reduced tumour blood vessel number and diameter. The reduced growth of these tumours was also reflected in the reduced (by 60-70%) infiltration of myeloid suppressor cells. Encouragingly VAP-1 deficiency had no effect on vessel or lymph formation in normal tissue.

For the above reasons, it is expected that inhibition of SSAO will reduce the levels of pro-inflammatory enzyme products (aldehydes, hydrogen peroxide and ammonia) whilst also decreasing the adhesive capacity of immune cells and correspondingly their activation and final extra-vasation. Diseases where such an activity is expected to be therapeutically beneficial include all diseases where immune cells play a prominent role in the initiation, maintenance or resolution of the pathology, such inflammatory diseases and immune/autoimmune diseases. Examples of such diseases include multiple sclerosis, arthritis and vasculitis.

WO2010/031789 (the content of which is herein incorporated by reference) discloses a promising class of SSAO inhibitor compounds, especially promising is Example 16, which is the free base of (3*S*)-Tetrahydrofuran-3-yl (4*S*)-4-isopropyl-1,4,6,7-tetrahydro-5H-imidazo[4,5-c]pyridine-5-carboxylate, and has the following structure:

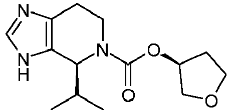

Pain is an unpleasant condition which may interfere with a person's quality of life. An unmet medical need exists for new treatments for pain.

### Summary of the invention

Following extensive investigations, it has been found that (3*S*)-Tetrahydrofuran-3-yl (4*S*)-4-isopropyl-1,4,6,7-tetrahydro-5H-imidazo[4,5-c]pyridine-5-carboxylate is surprisingly effective in the treatment of pain.

### Brief Description of the Drawings

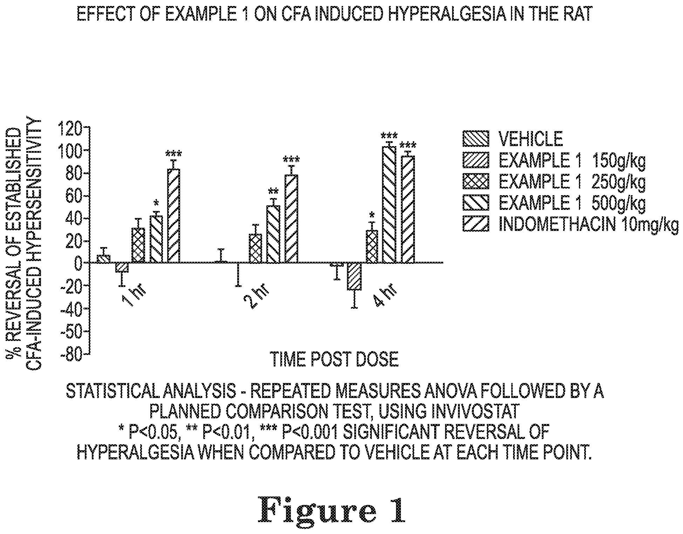

**Figure 1** shows the effect of (3*S*)-Tetrahydrofuran-3-yl (4*S*)-4-isopropyl-1,4,6,7-tetrahydro-5H-imidazo[4,5-c]pyridine-5-carboxylate in the CFA induced thermal hyperalgesia (pain) model.

### Definitions

The term 'pain' as used herein includes inflammatory pain. In an embodiment the pain is inflammatory pain.

"Treatment" as used herein includes prophylaxis of the named disorder or condition, or amelioration or elimination of the disorder once it has been established.

"An effective amount" refers to an amount of a compound that confers a therapeutic effect on the treated subject. The therapeutic effect may be objective (i.e., measurable by some test or marker) or subjective (i.e., subject gives an indication of or feels an effect).

"Pharmaceutically acceptable" means being useful in preparing a pharmaceutical composition that is generally safe, non-toxic and neither biologically nor otherwise undesirable and includes being useful for veterinary use as well as human pharmaceutical use.

Unless stated to the contrary, the term "(3*S*)-Tetrahydrofuran-3-yl (4*S*)-4-isopropyl-1,4,6,7-tetrahydro-5H-imidazo[4,5-c]pyridine-5-carboxylate" as used in connection with the crystalline salt form described herein includes a mixture of the (3S,4S) and (3R,4R) enantiomers. In an embodiment (3*S*)-Tetrahydrofuran-3-yl (4*S*)-4-isopropyl-1,4,6,7-tetrahydro-5H-imidazo[4,5-c]pyridine-5-carboxylate, and salts thereof, has an absolute purity of >95%, preferably >99%, more preferably >99.5%. In an embodiment (3*S*)-Tetrahydrofuran-3-yl (4*S*)-4-isopropyl-1,4,6,7-tetrahydro-5H-imidazo[4,5-c]pyridine-5-carboxylate means the (3S,4S) enantiomer having an enantiomeric purity of >95%, preferably >99%, more preferably >99.5%. In an embodiment (3*S*)-Tetrahydrofuran-3-yl (4*S*)-4-isopropyl-1,4,6,7-tetrahydro-5H-imidazo[4,5-c]pyridine-5-carboxylate has a diastereoisomeric purity of >95%, preferably >99%, more preferably >99.5%.

### Detailed description of the invention

(3*S*)-Tetrahydrofuran-3-yl (4*S*)-4-isopropyl-1,4,6,7-tetrahydro-5H-imidazo[4,5-c]pyridine-5-carboxylate may be used as such or in the form of a pharmaceutically acceptable salt. Salts include pharmaceutically acceptable salts, for example acid addition salts derived from inorganic or organic acids, such as hydrochlorides, hydrobromides, p-toluenesulphonates, phosphates, sulphates, perchlorates, acetates, trifluoroacetates, propionates, citrates, malonates, succinates, lactates, oxalates, tartrates and benzoates. For a review on salts, see Handbook of Pharmaceutical Salts: Properties. Selection, and Use by Stahl and Wermuth (Wiley-VCH, Weinheim, Germany, 2002).

Salts may also be formed with bases. Such salts include salts derived from inorganic or organic bases, for example alkali metal salts such as magnesium or calcium salts, and organic amine salts such as morpholine, piperidine, dimethylamine or diethylamine salts. A particular salt is the mesylate salt. An alternative salt is the sulphate salt, which exists as a hydrate; In an embodiment the hydrate is (3*S*)-Tetrahydrofuran-3-yl (4*S*)-4-isopropyl-1,4,6,7-tetrahydro-5H-imidazo[4,5-c]pyridine-5-carboxylate sulphate 1.5 H₂O.

A typical dosage is 2 to 20 mg/kg, administered one or more times per day or by continuous infusion A typical total daily dosage for a human is 1 to 2000mg/day, preferably from 200 to 2000mg/day, more preferably from 500 to 2000mg/day.

(3*S*)-Tetrahydrofuran-3-yl (4*S*)-4-isopropyl-1,4,6,7-tetrahydro-5H-imidazo[4,5-c]pyridine-5-carboxylate may be administered in a variety of dosage forms. Thus, it can be administered orally, for example as a tablet, a capsule, a troche, a lozenge, an aqueous or oily suspension, a dispersible powder or granule. The drug is preferably administered via the oral route. It will be understood, however, that the specific dose level for any particular patient will depend upon a variety of factors including the age, body weight, general health, sex, diet, time of administration, drug combination and the severity of the particular condition undergoing therapy.

A pharmaceutical composition containing the active ingredient may be in any suitable form, for example aqueous or non-aqueous solutions or suspensions, dispersible powders or granules, transdermal or transmucosal patches, creams, ointments or emulsions.

The pharmaceutical composition may be in the form of a sterile injectable aqueous or non-aqueous (e.g. oleaginous) solution or suspension. The sterile injectable preparation may also be in a sterile injectable solution or suspension in a non-toxic parenterally-acceptable diluent or solvent, for example as a solution in 1,3-butanediol. Among the acceptable vehicles and solvents that may be employed are water, phosphate buffer solution, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables. Suspensions may be formulated according to the known art using those suitable dispersing or wetting agents and suspending agents.

Aqueous suspensions contain the active ingredient in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents such as a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Non-aqueous (i.e. oily) suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are known.

The active agent may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical delivery, transdermal and transmucosal patches, creams, ointments, jellies, solutions or suspensions may be employed. For sub-lingual delivery, fast dissolving tablet formulations may be used, as well as a number of the presentations described above. For oral administration, the drug may be administered as tablets, capsules or liquids.

The following study provides evidence on which the present invention is based.
(3*S*)-Tetrahydrofuran-3-yl (4*S*)-4-isopropyl-1,4,6,7-tetrahydro-5H-imidazo[4,5-c]-pyridine-5-carboxylate was investigated (Figure 1) in the CFA thermal hyperalgesia model, which is an established model for inflammatory pain. (4*S*)-4-isopropyl-1,4,6,7-tetrahydro-5H-imidazo[4,5-c]pyridine-5-carboxylate was found to be effective in a dose-dependent manner, and comparably in efficacy to the gold standard benchmark indomethacin. In more detail:
*Evaluation of (3S)-Tetrahydrofuran-3-yl (4S)-4-isopropyl-1,4,6,7-tetrahydro-5H-imidazo[4,5-c]pyridine-5-carboxylate mesylate salt on CFA (Complete Freunds Adjuvant) induced hypersensitivity in rat*
Assessment of the anti-hyperalgesic properties of *(3S)*-tetrahydrofuran-3-yl (4S)-4-isopropyl-1,4,6,7-tetrahydro-5H-imidazo[4,5-c]pyridine-5-carboxylate mesylate salt was determined through measurement of weight bearing following CFA induced hypersensitivity. Naive rats distribute their body weight equally between the two hind paws. However, when the injected (left) hind paw is painful, the weight is redistributed so that less weight is put on the affected paw (decrease in weight bearing on injured paw). Weight bearing through each hind limb was measured using a rat incapacitance tester (Linton Instruments, UK). Rats were placed in the incapacitance tester with the hind paws on separate sensors and the average force exerted by both hind limbs was recorded over 4 seconds. The injection of CFA also induces an oedema that can be assessed by paw volume; this is measured using a plethysmometer. The rat's hind paw is placed into the cylinder containing a solution and the volume of displaced liquid determines the paw volume.
Naive male, Sprague Dawley rats were acclimatised with food and water available ad libitum. Habituation to the incapacitance tester was performed. Baseline weight bearing and paw volume recordings were taken prior to induction of insult. Inflammatory hypersensitivity was induced by intraplantar injection of CFA (100ul of 1mg/ml solution) into the left hind paw. A pre-treatment weight bearing and paw volume measurement was taken to assess hypersensitivity 23 hours post-CFA. Animals were then ranked and randomised according to CFA window in a Latin square design. *(3S)*-Tetrahydrofuran-3-yl (4*S*)-4-isopropyl-1,4,6,7-tetrahydro-5H-imidazo[4,5-c]pyridine-5-carboxylate mesylate salt was then given at 150, 250 and 500mg/kg p.o. (dosed in water at 2mL/Kg, pH ∼5-6), alongside vehicle and reference group (indomethacin), n=9-10 per group. Weight bearing was assessed in all groups 1, 2 and 4 hours post compound administration. Paw volume was assessed 4 hours post compound administration. Data was analysed by comparing treatment groups to control group at each time point.
Weight bearing (g) readings were taken for both right and left hind paws and the difference calculated. Data is expressed as % reversal of the hypersensitivity to pain, (post dose reading - pre dose reading)/ (naïve reading - pre dose reading) x 100, where naive weight bearing difference - pre dose weight bearing difference is defined as the CFA window to be reversed. Statistical analysis was conducted by means of repeated measures ANOVA followed by Planned comparison test using InVivoStat (invivostat.co.uk), (p<0.05 considered significant).
(*3S*)-Tetrahydrofuran-3-yl (4S)-4-isopropyl-1,4,6,7-tetrahydro-5H-imidazo[4,5-c]-pyridine-5-carboxylate mesylate salt at 150mg/kg showed no significant reversal of hypersensitivity at any time point, 250mg/kg showed a significant reversal at 4 hours, however 500mg/kg was effective at all time points with maximum effect seen at 4 hours post dose. No effect was seen on paw volume with (*3S*)-tetrahydrofuran-3-yl (*4S*)-4-isopropyl-1,4,6,7-tetrahydro-5H-imidazo[4,5-c]pyridine-5-carboxylate mesylate salt.

### Synthesis

The following abbreviations have been used:
- Aq: Aqueous
- DCM: Dichloromethane
- DIPEA: Diisopropylethylamine
- ee: Enantiomeric excess
- ES⁺: Electrospray
- EtOAc: Ethyl acetate
- h: Hour(s)
- HPLC: High performance liquid chromatography
- HRMS: High resolution mass spectrometry
- LCMS: Liquid chromatography mass spectrometry
- M: Molar
- MeOH: Methanol
- [MH⁺]: Protonated molecular ion
- min: Minutes
- RP: Reverse phase
- MS: Mass spectrometry
- R_{T}: Retention time
- sat: Saturated
- THF: Tetrahydrofuran
- TFA: Trifluoroacetic acid

### Experimental Methods

All reagents were commercial grade and were used as received without further purification, unless otherwise specified. Reagent grade solvents were used in all cases.

Analytical LCMS was performed on a Waters ZQ mass spectrometer connected to an Agilent 1100 HPLC system. Analytical HPLC was performed on an Agilent 1100 system. High-resolution mass spectra (HRMS) were obtained on an Agilent MSD-TOF connected to an Agilent 1100 HPLC system. During the analyses the calibration was checked by two masses and automatically corrected when needed. Spectra are acquired in positive electrospray mode. The acquired mass range was m/z 100-1100. Profile detection of the mass peaks was used. Flash chromatography was performed on either a CombiFlash Companion system equipped with RediSep silica columns or a Flash Master Personal system equipped with Strata SI-1 silica gigatubes. Reverse Phase HPLC was performed on a Gilson system (Gilson 322 pump with Gilson 321 equilibration pump and Gilson 215 autosampler) equipped with Phenomenex Synergi Hydro RP 150 x 10 mm, YMC ODS-A 100/150 x 20 mm or Chirobiotic T 250 x 10 mm columns. Reverse phase column chromatography was performed on a Gilson system (Gilson 321 pump and Gilson FC204 fraction collector) equipped with Merck LiChroprep® RP-18 (40-63 µm) silica columns. The compounds were automatically named using ACD 6.0. All compounds were dried in a vacuum oven overnight.

Analytical HPLC and LCMS data were obtained with:
System A: Phenomenex Synergi Hydro RP (C18, 30 x 4.6 mm, 4 µm), gradient 5-100% CH₃CN (+0.085% TFA) in water (+0.1% TFA), 1.5 mL/min, with a gradient time of 1.75 min, 200 nm, 30 °C; or
System B: Phenomenex Synergi Hydro RP (C18, 150 x 4.6 mm, 4 µm), gradient 5-100% CH₃CN (+0.085% TFA) in water (+0.1% TFA), 1.5 mL/min with a gradient time of 7 min, 200 nm, 30 °C.

Chiral HPLC data were obtained with:
System C: Chirobiotic V polar ionic mode (150 x 4.6 mm), 70% MeOH in 10 mM aq ammonium formate buffer, 1.0 mL/min, over 10 min, 200 nm, 30 °C.

### INTERMEDIATE 1

### 4-Isopropyl-4,5,6,7-tetrahydro-1H-imidazo[4,5-c]pyridine hydrochloride

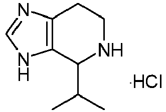

Histamine dihydrochloride (61.9 g, 336 mmol) was dissolved in a solution of NaOH (33.6 g, 841 mmol) in water (125 mL) and MeOH (500 mL), and isobutyraldehyde (61.4 mL, 672 mmol) was added. The reaction mixture was heated under reflux at 80 °C for 24 h, cooled to room temperature, the pH was adjusted to 7 with 1 M aq HCI solution (250 mL) and the solvents were removed *in vacuo.* The residue was dissolved in warm MeOH (300 mL), allowed to stand for 1h, filtered and the solvents were removed *in vacuo.* The residue was stirred in MeOH (50 mL) and acetone (400 mL) for 2 h and was cooled to 4 °C for 2 h. The resulting precipitate was filtered and washed with acetone (100 mL) to give 4-isopropyl-4,5,6,7-tetrahydro-1H-imidazo[4,5-c]pyridine hydrochloride (33.0 g, 48.7%) as a white solid.
Analytical LCMS: purity >90% (System A, R_{T} = 0.51 min), ES⁺: 166.4 [MH]⁺.

### INTERMEDIATE 2

### 4-Nitrophenyl 4-isopropyl-1,4,6,7-tetrahydro-5H-imidazo[4,5-c]pyridine-5-carboxylate

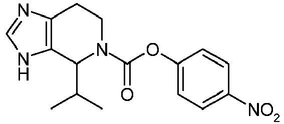

Intermediate 1 (2.78 g, 8.28 mmol, 60% pure) and DIPEA (5.27 mL, 30.3 mmol) were dissolved in DCM (100 mL). The reaction mixture was cooled to 0 °C and 4-nitrophenyl chloroformate (4.07 g, 20.2 mmol) was added. The reaction mixture was stirred at room temperature for 18 h. The reaction mixture was washed with sat aq NaHCO₃ solution (5 x 100 mL), dried (MgSO₄) and the solvents were removed *in vacuo* to give 4-nitrophenyl 4-isopropyl-1,4,6,7-tetrahydro-5H-imidazo[4,5-c]pyridine-5-carboxylate (5.28 g, crude) as a yellow gum.
Analytical HPLC: purity 41% (System B, R_{T} = 4.70 min); Analytical LCMS: purity 86% (System A, R_{T} = 1.70 min), ES⁺: 331.0 [MH]⁺.

### (3S)-Tetrahydrofuran-3-yl (4S)-4-isopropyl-1,4,6,7-tetrahydro-5H-imidazo[4,5-c]pyridine-5-carboxylate

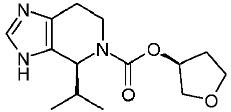

NaH (0.40 g, 10.0 mmol, 60% dispersion in mineral oil) was suspended in anhydrous THF (20 mL), cooled to 0 °C and (S)-3-hydroxytetrahydrofuran (0.88 g, 0.68 mL, 10.0 mmol) was added. The suspension was stirred at 0 °C for 30 min then added to a solution of Intermediate 2 (3.30 g, 10.0 mmol, 70% pure) in THF (60 mL) and the reaction mixture was stirred at room temperature. Two additional such portions of NaH and (*S*)-3-hydroxytetrahydrofuran in THF were added after 5 and 29 h, respectively. After 2 d the reaction mixture was quenched with water (10 mL) and the solvents were removed *in vacuo.* The residue was dissolved in EtOAc (100 mL), washed with 1 M aq Na₂CO₃ solution (4 x 100 mL), dried (MgSO₄) and the solvents were removed *in vacuo.* The residue was purified by column chromatography (normal phase, 20 g, Strata Sl-1, silica gigatube, DCM (200 mL) followed by 2%, 4% and 5% MeOH in DCM (200 mL each)) and reverse phase HPLC (YMC ODS-A 100 x 20 mm, 5 µm, 25 mL/min, gradient 30% to 60% (over 7 min) then 100% (3 min) MeOH in 10% MeOH/water) to give (3*S*)-tetrahydrofuran-3-yl 4-isopropyl-1,4,6,7-tetrahydro-5H-imidazo[4,5-c]pyridine-5-carboxylate (34.8 mg, 1.1%) as a white solid. Analytical HPLC: purity 100% (System B, R_{T} = 3.63 min); Analytical LCMS: purity 100% (System B, R_{T} = 4.01 min), ES⁺: 280.1 [MH]⁺.

(3*S*)-Tetrahydrofuran-3-yl-4-isopropyl-1,4,6,7-tetrahydro-5H-imidazo[4,5-c]pyridine-5-carboxylate (39.91 mg) was dissolved in 10 mM ammonium formate buffer and MeOH (2 mL, 1:1) and purified twice by reverse phase chiral HPLC (Chirobiotic T 250 x 10 mm, 3 mL/min, isocratic run 70% MeOH in 10 mM ammonium formate buffer (40 min), pH 7.4) to give a single diastereoisomer, (3*S*)-tetrahydrofuran-3-yl (4*S*)-4-isopropyl-1,4,6,7-tetrahydro-5H-imidazo[4,5-c]pyridine-5-carboxylate (6.90 mg, 99% ee).
Analytical HPLC: purity 100% (System B, R_{T} = 3.63 min); Chiral HPLC: purity 99.5% (System C, R_{T} = 2.22 min); Analytical LCMS: purity 100% (System B, R_{T} = 3.90 min), ES⁺: 280.1 [MH]⁺; HRMS calculated for C₁₄H₂₁N₃O₃: 279.1583, found 279.1571.

### (3S)-Tetrahydrofuran-3-yl(4S)-4-isopropyl-1,4,6,7-tetrahydro-5H-imidazo[4,5-c]pyridine-5-carboxylate, Methananesulfonic acid salt

### (Example 1)

(3S)-Tetrahydrofuran-3-yl (4*S*)-4-isopropyl-1,4,6,7-tetrahydro-5H-imidazo[4,5-c]-pyridine-5-carboxylate free base (460mg, 1.65mmol) was dissolved in EtOAc (10mL) at room temperature to give a clear colourless solution. Methanesulphonic acid (107uL) was added portion-wise with gentle heating. The solution was allowed to cool to room temperature overnight. The resulting crystals were collected by filtration, washed with EtOAc (2 x 10mL) and dried overnight at 40°C *in vacuo.* (3*S*)-Tetrahydrofuran-3-yl (4*S*)-4-isopropyl-1,4,6,7-tetrahydro-5H-imidazo[4,5-c]pyridine-5-carboxylate mesylate salt was obtained with a 99% yield (615mg) as a white crystalline solid. HPLC: Retention time 2.27min, purity 99.5%. Melting point: 189°C. LCMS: Retention time 4.19min, ES⁺ 280.0 [MH]⁺, 100% purity. Chiral HPLC: Retention time 3.70min, >99.5% de. ¹H NMR (400MHz, CDCl₃). ^{δ}_{H} 8.72 (1H, m, NHCHNH⁺), 5.29 (1H, m, OCH), 5.05 (0.5H, d, *J* 8.4Hz, CCHN), 4.89 (0.5H, d, *J* 7.6Hz, CCHN), 4.59 (0.5H, m, NCH_{A}CH_{B}), 4.39 (0.5H, m, NCH_{A}CH_{B}), 3.97-3.85 (4H, m, CH₂OCH₂), 3.20 (1H, m, NCH_{A}CH_{B}), 2.89 (3H, s, CH₃SO₃⁻), 2.89-2.72 (2H, m, CCH₂CH₂N), 2.23-2.07 (3H, m, CH(CH₃)₂, OCH₂CH₂), 1.16 (3H, d, *J*6.4Hz, CH₃) and 1.06-0.96 (3H, m, CH₃).

## Claims

1. (3*S*)-Tetrahydrofuran-3-yl (4*S*)-4-isopropyl-1,4,6,7-tetrahydro-5H-imidazo[4,5-c]-pyridine-5-carboxylate and hydrates and pharmaceutically acceptable salts thereof, for use in the treatment of pain.

2. A pharmaceutical composition comprising a compound according to claim 1, and one or more suitable excipients, for use in the treatment of pain.

3. A compound for use according to claim 1 wherein the pain is inflammatory pain.

4. A compound for use according to claim 1 wherein the pharmaceutically acceptable salt is the mesylate.

5. A compound for use according to claim 1 wherein the pharmaceutically acceptable salt is the sulphate, or a hydrate thereof.

## Patentansprüche

1. (3*S*)-Tetrahydrofuran-3-yl (4*S*)-4-Isopropyl-1,4,6,7-tetrahydro-5H-imidazo[4,5-c]-pyridin-5-carboxylat und Hydrate und pharmazeutisch unbedenkliche Salze davon, zur Verwendung bei der Behandlung von Schmerzen.

2. Pharmazeutische Zusammensetzung, umfassend eine Verbindung nach Anspruch 1 und einen oder mehrere geeignete Exzipienten, zur Verwendung bei der Behandlung von Schmerzen.

3. Verbindung zur Verwendung nach Anspruch 1, wobei die Schmerzen Entzündungsschmerzen sind.

4. Verbindung zur Verwendung nach Anspruch 1, wobei das pharmazeutisch unbedenkliche Salz das Mesylat ist.

5. Verbindung zur Verwendung nach Anspruch 1, wobei das pharmazeutisch unbedenkliche Salz das Sulfat, oder ein Hydrat davon, ist.

## Revendications

1. (4*S*)-4-isopropyl-1,4,6,7-tétrahydro-5H-imidazo[4,5-c]-pyridine-5-carboxylate de (3*S*)-tétrahydrofuran-3-yle et hydrates et sels pharmaceutiquement acceptables de celui-ci, à utiliser dans le traitement de la douleur.

2. Composition pharmaceutique comprenant un composé selon la revendication 1, et un ou plusieurs excipients adéquats, à utiliser dans le traitement de la douleur.

3. Composé à utiliser selon la revendication 1, dans lequel la douleur est une douleur inflammatoire.

4. Composé à utiliser selon la revendication 1, dans lequel le sel pharmaceutiquement acceptable est le mésylate.

5. Composé à utiliser selon la revendication 1, dans lequel le sel pharmaceutiquement acceptable est le sulfate, ou un hydrate de celui-ci.
